# EUROPEAN PATENT APPLICATION

(11) **EP 2 965 697 A1**
(43) Date of publication of application: **13.01.2016**
(21) Application number: 13877080.5
(22) Date of filing: 08.03.2013
(51) Int. Cl.: A61B 17/28

(54) **INTRACORPOREAL INTRODUCTION INSTRUMENT**

(71) Applicant: Japanese Organization for Medical Device Development, Inc., Tokyo 103-0023 (JP)
(72) Inventor: OHKI, Takao, Tokyo 105-0002 (JP)
(74) Representative: Pitchford, James Edward
(86) International application number: PCT/JP2013/001521
(87) International publication number: WO 2014/136147

(57) **Abstract**

The present invention addresses the problem of providing an intracorporeal introduction instrument capable of reducing patient and medical practitioner hardship by simplifying surgical procedures and shortening surgery time. An intracorporeal introduction instrument (1), which has grasping sections (3a, 3b) at one end of a pair of rod-shaped members, handle sections (4a, 4b) at the other end, and movement-transmitting sections (5a, 5b) between the grasping sections and the handle sections, respectively, and which opens and closes the grasping sections via the movement-transmitting sections according to the opening and closing of the handle sections. The intracorporeal introduction instrument is characterized in that the movement-transmitting sections form a double lever mechanism, which transmits opening and closing movements of the handle sections as opening and closing movements of the grasping sections.

## Description

### Technical Field

The present invention relates to an intracorporeal introduction instrument, and especially relates to an intracorporeal introduction instrument used to form a tunnel in a living body, and to introduce a medium such as an artificial blood vessel into the living body.

### Background Art

When an artery of a lower limb or the like is clogged, a bypass operation to insert artificial blood vessels in front and behind the clogged portion of the artery and replace the clogged portion with the artificial blood vessels is performed, and when a varix is caused in a lower limb or the like, a bypass operation to separate and remove the varix, and then insert an artificial blood vessel and replace the varix with the artificial blood vessel is performed.

In these bypass operations, for example, a long rod-like instrument called Gore tunneler is sometimes used. The Gore tunneler includes, for example, a handle section held by an operator, a rod section extending ahead of the handle section, a sleeve section into which the rod section is inserted, and a cap section screwed into a tip of the sleeve section. The Gore tunneler is used as follows, for example. First, the Gore tunneler assembled such that the rod section is inserted to the sleeve section having a tip with which the cap section is screwed is inserted through one incised inlet in a body surface along a subcutaneous in the body, and is caused to penetrate the other incised outlet in the body surface. The length of the penetration exceeds 20 cm depending on a portion. Next, the cap section provided on the tip of the Gore tunneler exposed from the body surface is removed, and an artificial blood vessel is fixed to a tip of the rod section with a string, and then the rod section is pulled in an opposite direction to an insertion direction and the artificial blood vessel is inserted into the sleeve. After the inserted artificial blood vessel and the rod section are cut apart, the sleeve section is pulled out, and the artificial blood vessel is arranged in a desired portion of the living body.

For example, Patent Literature 1 describes "a guide tool of an artificial blood vessel or the like, in which a curved section having a fixed bending angle is formed at an arbitrary position of a hollow tube that enables the artificial blood vessel or the like to be inserted/removed, and which includes a substantially circular or pyramid-shaped guide head attachably/detachably included in one end portion" (claim 1 of Patent Literature 1), as an object of "providing an easy-to-handle and safe guide tool of an artificial blood vessel or the like, with a simple structure" (Paragraph 0004 of Patent Literature 1).

Further, Patent Literature 2 describes "a tunnel device including a unit that supplies tissue separation energy to a tissue cell adjacent to the tip chip (112, 912) ..." (claim 1 of Patent Literature 2), as an object of providing a tunneler that enables transplant with a smaller tissue injury than a tissue injury caused by a conventional tunneler.

### Citation List

### Patent Literatures

Patent Literature 1: JP 09-220244 A
Patent Literature 2: JP 2008-508949 W

### Summary of Invention

### Technical Problem

In the conventional so-called tunnelers, operations such as removing the cap section attachably/detachably provided on the tip after inserting the tunneler into the body, and fixing the artificial blood vessel with a string are required, and the operations take time and labor, and become a significant burden on patients and medical professionals.

The present invention has been made to solve the problem, and an object is to provide an intracorporeal introduction instrument that can reduce the burden on the patients and medical professionals, by simplifying a surgical operation and shortening a surgical time.

### Solution to Problem

In order to achieve the object described above, provided is:
(1) an intracorporeal introduction instrument including:
   grasping sections at one end of a pair of rod-like members;
   handle sections at the other end of the pair of rod-like members; and
   movement transmission sections between the grasping sections and the handle sections,
   the grasping sections configured to be opened and closed through the movement transmission sections according to opening and closing of the handle sections, wherein
   the movement transmission sections form a double lever mechanism that transmits opening and closing movement of the handle sections, as opening and closing movement of the grasping sections.

As preferred aspects of (1), provided are:
(2) an opening and closing direction of the handle sections and an opening and closing direction of the grasping sections are perpendicular to each other;
(3) the intracorporeal introduction instrument according to (1) or (2), wherein the movement transmission sections include a gently bent curved section;
(4) the intracorporeal introduction instrument according to any one of (1) to (3), wherein an external form of the grasping sections is an approximately truncated pyramid shape when the pair of grasping sections is in a closed state; and
(5) the intracorporeal introduction instrument according to any one of (1) to (4), wherein a length L1 of the movement transmission sections in a longitudinal direction is 170 to 350 mm, a length L2 of the grasping sections in the longitudinal direction is 10 to 20 mm, a length D1 of the movement transmission sections in a width direction perpendicular to the longitudinal direction is 7 to 13 mm, a length D2 at base end portions of the grasping sections in the width direction is 7 to 13 mm, a total thickness D3 at a position of 20% of the length L2 from a tip of the grasping sections of when the pair of grasping sections is in the closed state is 4 to 8 mm, and a length D4 in a direction perpendicular to the total thickness D3 at the position is 5 to 11 mm.

### Advantageous Effects of Invention

An intracorporeal introduction instrument according to the present invention includes grasping sections at one end of a pair of rod-like members and handle sections at the other end of the pair of rod-like members, and the grasping sections are opened/closed according to opening/closing of the handle sections. Therefore, it is not necessary to perform operations such as removing a cap section provided on a tip and fixing an artificial blood vessel with a string, like the conventional so-called tunneler, after the intracorporeal introduction instrument is introduced into a living body and a tunnel or a pathway is formed. Further, a medium such as the artificial blood vessel is grasped with the grasping sections and pulled back, so that the medium such as the artificial blood vessel can be easily introduced into the body. Therefore, the surgical operation can be simplified and the surgical time can be shortened, whereby the burden on the patients and the medical professionals can be reduced.

Further, the structure is simpler than that of the conventional so-called tunneler, and thus washing and sterilization are easy, and the instrument can be easily managed. Therefore, according to the present invention, management of medical instruments can be rationalized, and total cost can be decreased, accordingly.

### Brief Description of Drawings

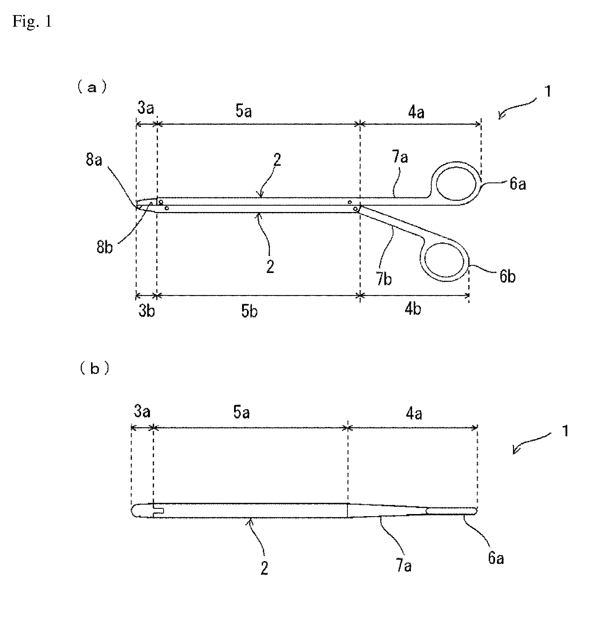

Figs. 1(a) and 1(b) are schematic explanatory diagrams of an intracorporeal introduction instrument, illustrating an embodiment of an intracorporeal introduction instrument according to the present invention. Fig. 1(a) is a top-view schematic explanatory diagram of an intracorporeal introduction instrument, illustrating an embodiment of an intracorporeal introduction instrument according to the present invention, and Fig. 1(b) is a side-view schematic explanatory diagram of the intracorporeal introduction instrument illustrated in Fig. 1(a).

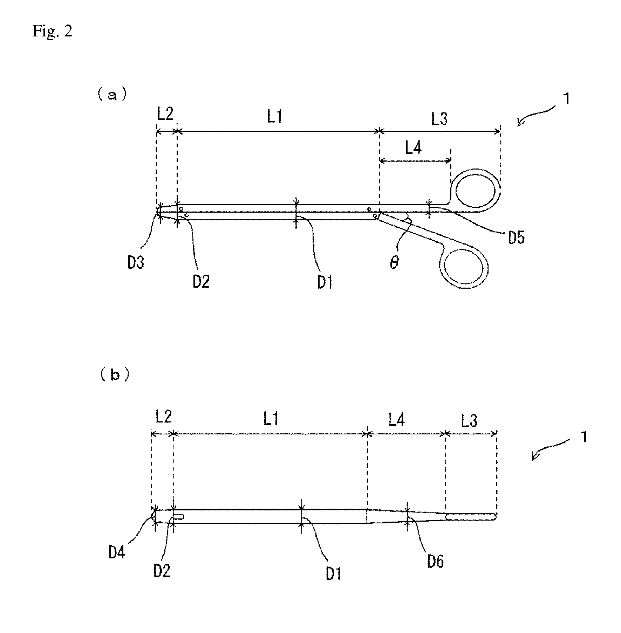

Figs. 2(a) and 2(b) are schematic explanatory diagrams illustrating dimensions of the intracorporeal introduction instrument illustrated in Figs. 1(a) and 1(b). Fig. 2(a) is a top-view schematic explanatory diagram of an intracorporeal introduction instrument, illustrating an embodiment of an intracorporeal introduction instrument according to the present invention, and Fig. 2(b) is a side-view schematic explanatory diagram of the intracorporeal introduction instrument illustrated in Fig. 2(a).

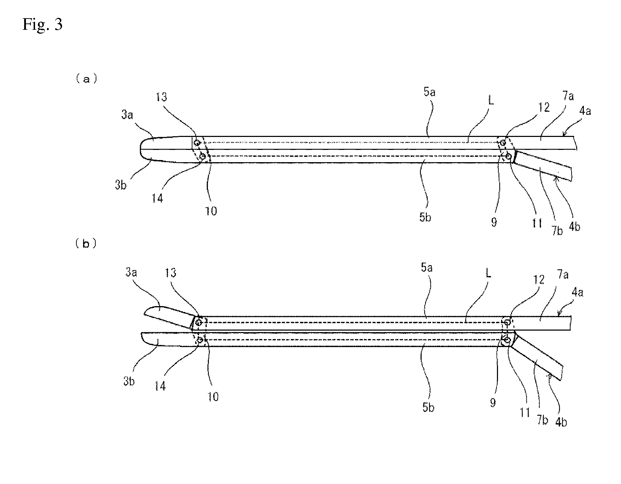

Figs. 3(a) and 3(b) are schematic diagrams of principal parts, illustrating enlarged movement transmission sections and grasping sections. Fig. 3(a) is a schematic diagram of principal parts, illustrating enlarged movement transmission sections and grasping sections when the grasping sections are in a closed state, and Fig. 3(b) is a schematic diagram of principal parts, illustrating enlarged movement transmission sections and grasping sections when the grasping sections are in an opened state.

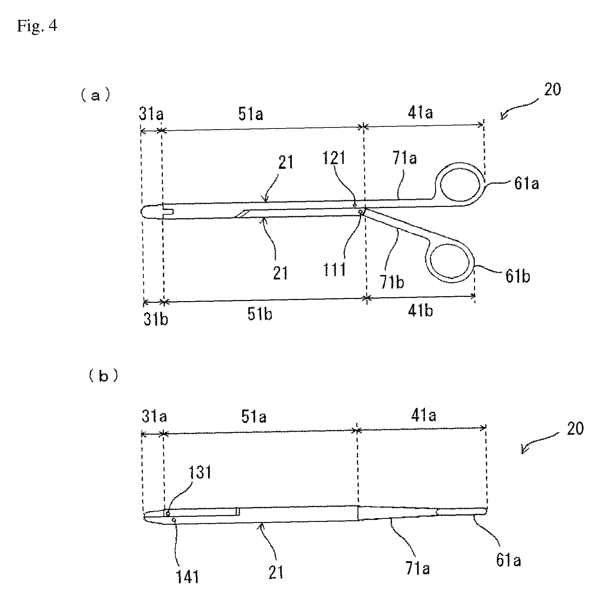

Figs. 4(a) and 4(b) are schematic explanatory diagrams of an intracorporeal introduction instrument, illustrating another embodiment of an intracorporeal introduction instrument according to the present invention. Fig. 4(a) is a top-view schematic explanatory diagram of an intracorporeal introduction instrument, illustrating another embodiment of an intracorporeal introduction instrument according to the present invention, and Fig. 4(b) is a side-view schematic explanatory diagram of the intracorporeal introduction instrument illustrated in Fig. 4(a).

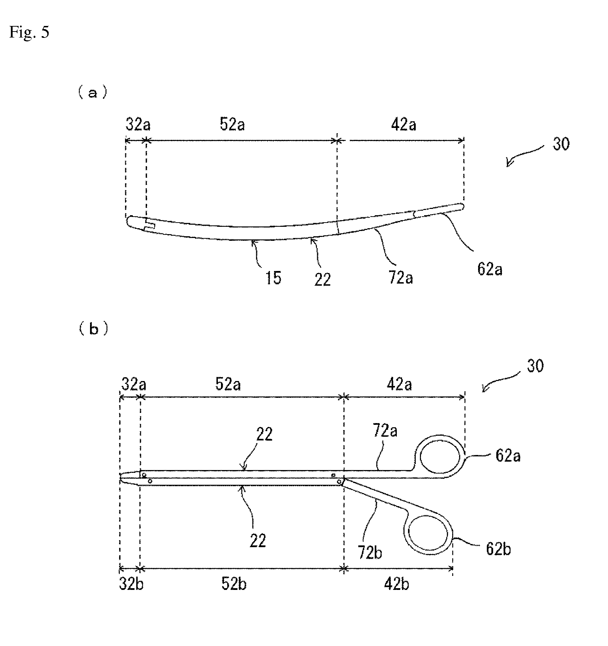

Figs. 5(a) and 5(b) are schematic explanatory diagrams of an intracorporeal introduction instrument, illustrating another embodiment of an intracorporeal introduction instrument according to the present invention. Fig. 5(a) is a side-view schematic explanatory diagram of an intracorporeal introduction instrument, illustrating another embodiment of an intracorporeal introduction instrument according to the present invention, and Fig. 5(b) is a top-view schematic explanatory diagram of the intracorporeal introduction instrument illustrated in Fig. 5(a).

### Description of Embodiments

An intracorporeal introduction instrument of the present invention is used to arrange a medium in a tunnel or a pathway, by being introduced into a living body through an incised inlet in a body surface, forming the tunnel or the pathway having a predetermined size, especially in a subcutaneous in the living body, and leading a tip portion of the intracorporeal introduction instrument through an incised outlet in the body surface, and then grasping and pulling back the medium to be arranged in the formed tunnel or pathway.

The medium is not especially limited as long as it needs to be arranged in the living body, and examples of the medium include a biological vessel such as a blood vessel of the living body, and an artificial vessel such as an artificial blood vessel. The thickness and the length of the tunnel or the pathway are appropriately set according to the thickness and the length of the medium, a portion where the medium is arranged, and the like, and dimensions of the intracorporeal introduction instrument of the present invention are also appropriately set according to the thickness and the length of the medium, the portion where the medium is arranged, and the like.

Hereinafter, an example of the intracorporeal introduction instrument used when an artificial blood vessel is arranged in a lower limb will be described as an example of an intracorporeal introduction instrument according to the present invention with reference to the drawings. Fig. 1(a) is a top-view schematic explanatory diagram of an intracorporeal introduction instrument, illustrating an embodiment of an intracorporeal introduction instrument according to the present invention, and Fig. 1(b) is a side-view schematic explanatory diagram of the intracorporeal introduction instrument illustrated in Fig. 1(a).

As illustrated in Figs. 1(a) and 1(b), an intracorporeal introduction instrument 1 of the present embodiment includes grasping sections 3a and 3b at one end of a pair of rod-like members 2, handle sections 4a and 4b at the other end of the pair of rod-like members 2, and movement transmission sections 5a and 5b between the grasping sections 3a and 3b, and the handle sections 4a and 4b. The grasping sections 3a and 3b are opened and closed according to opening and closing of the handle sections 4a, and 4b through the movement transmission sections 5a and 5b.

The rod-like members 2 can be formed of a material typically used as a material of a forceps, a pair of surgical scissors, or a so-called tunneler, of medical use, and are formed of titanium, a titanium alloy such as Ti6Al4, or stainless steel, for example.

The handle sections 4a and 4b are portions that open and close the grasping sections 3a and 3b, by being grasped, and opened and closed by the medical professional. The handle sections 4a and 4b include ring-like ring sections 6a and 6b into which fingers are inserted, and arm sections 7a and 7b extending from the ring sections 6a and 6b toward the movement transmission sections 5a and 5b. As illustrated in Figs. 2(a) and 2(b), a length L3 of the handle sections 4a and 4b in a direction into which the arm sections 7a and 7b extend can be appropriately set according to a portion used of the intracorporeal introduction instrument 1, and from a viewpoint of operability, and is 40 to 150 mm, for example.

One arm section 7a of the arm sections 7a and 7b linearly extends integrally with one movement transmission section 5a, when the pair of handle sections 4a and 4b are viewed from a top view, as illustrated in Fig. 1(a). The other arm section 7b is connected with the other movement transmission section 5b at a predetermined angle as a separate body. Therefore, when the grasping sections 3a and 3b and the handle sections 4a and 4b are in the closed state, the one arm section 7a and the other arm section 7b intersect with each other at a predetermined angle θ, for example, 10 to 20°, at end portions of the movement transmission sections 5a and 5b sides. A length L4 of the arm sections 7a and 7b in a longitudinal direction is not especially limited. However, the length L4 is 20 to 130 mm, for example, in consideration of the operability of the intracorporeal introduction instrument. Further, as cross section shapes of the arm sections 7a and 7b, a square shape with rounded corners, a circular shape, an elliptical shape, or the like can be employed. In the center of the length of the arm section 7a and 7b in the longitudinal direction, a length D5 perpendicular to the longitudinal direction, and in a direction into which the handle sections 4a and 4b are opened and closed is 3.5 to 7.5 mm, for example, 5.5 mm, and a length D6 in a direction perpendicular to the direction into which the handle sections 4a and 4b are opened and closed is 3.5 to 15 mm, for example, 10 mm. In the present embodiment, the arm section 7a and 7b may be a rod-like shape that gradually becomes thicker from the ring sections 6a and 6b side toward the movement transmission sections 5a and 5b, but does not change the thickness in the longitudinal direction. Note that it is favorable that all corners of an external surface of the intracorporeal introduction instrument 1 are rounded so as not to hurt a biological tissue.

The movement transmission sections 5a and 5b form a double lever mechanism that transmits opening and closing movement of the handle sections 4a and 4b, as opening and closing movement of the grasping sections 3a and 3b. Since the movement transmission sections 5a and 5b form the double lever mechanism, the shapes of the movement transmission sections 5a and 5b are almost unchanged in the opening and closing movement. In other words, the grasping sections 3a and 3b can be opened and closed in a state where the pair of movement transmission sections 5a and 5b is not substantially separated from each other. Therefore, when the medium such as the artificial blood vessel is grasped with the grasping sections 3a and 3b after the intracorporeal introduction instrument 1 is introduced into a living body, the grasping sections 3a and 3b can be opened and closed without substantially changing the external forms of the movement transmission sections 5a and 5b arranged in the living body.

Figs. 3(a) and 3(b) are schematic diagrams of principal parts, illustrating enlarged movement transmission sections. Fig. 3(a) illustrates the movement transmission sections of when the grasping sections are in the closed state, and Fig. 3(b) illustrates the movement transmission sections of when the grasping sections are in the opened state.

As illustrated in Figs. 3(a) and (b), one movement transmission section 5b and the other movement transmission section 5a are coupled with a first link 9 existing at a tip of one handle section 4b and a second link 10 existing at a base end of the other grasping section 3a through four pins from a first pin 11 to a fourth pin 14. In these movement transmission sections 5a and 5b, the first link 11 and the second link 12 function as levers, and the one movement transmission section 5b functions as a fixed link, so that the double lever mechanism is formed. As illustrated in Fig. 3(a), when the pair of grasping sections 3a and 3b is closed, being in contact with each other, a virtual line L connecting the first pin 11, the second pin 12, the third pin 13, the fourth pin 14, and the first pin 11 in that order forms a parallelogram. As illustrated in Fig. 3(b), when the pair of grasping sections 3a and 3b is opened, being separated from each other, the virtual line L forms a shape approximating a rectangular, and the distance between the pair of movement transmission sections 5a and 5b is slightly separated by about 0.1 to several mm. As described above, the external forms of the movement transmission sections 5a and 5b are almost unchanged due to the opening and closing of the grasping sections 3a and 3b.

Cross section shapes and the thickness of the movement transmission sections 5a and 5b can be made to a shape and thickness approximating the arm sections 7a and 7b, and can be appropriately set according to a portion used of the intracorporeal introduction instrument 1, and from a viewpoint of the operability and the like. As illustrated in Figs. 2(a) and 2(b), a length L1 of the movement transmission sections 5a and 5b in the longitudinal direction is 170 to 350 mm, for example, 260 mm, and a total thickness, that is, a length D1 of two directions of the movement transmission sections 5a and 5b in a width direction perpendicular to the longitudinal direction is 7 to 13 mm, for example, 10 mm.

Hereinafter, the double lever mechanism of the present embodiment will be described in more detail.

The one movement transmission section 5b is integrally connected with the one grasping section 3b in a linear manner, and functions as the fixed link. This movement transmission section 5b and the first link 9 existing at the end portion of the one handle section 4b are connected through the first pin 11, and the one handle section 4b is rotated around the first pin 11. An end portion of the first link 9, of an opposite side to the first pin 11, is connected with the other movement transmission section 5a through the second pin 12, and the other movement transmission section 5a swings according to the rotation of the one handle section 4b. The other movement transmission section 5a is integrally connected with the other handle section 4a in a linear manner, and is connected with the second link 10 existing at the base end of the other grasping section 3 a through the third pin 13. An end portion of the second link 10, of an opposite side to the third pin 13, is connected with the one movement transmission section 5b through the fourth pin 14. When the other movement transmission section 5a connected with the first link 9 through the second pin 12 swings, with the rotation of the one handle section 4b, the second link 10 swings with the swing, so that the other grasping section 3a is rotated around the fourth pin 14.

The first link 9 is provided, being tilted at a predetermined angle with respect to the arm section 7b, and the second link 10 is provided, being tilted at a predetermined angle with respect to the grasping section 3a at a predetermined angle. The first link 9 and the second link 10 are slightly longer than a total length of the widths of the movement transmission sections 5a and 5b, as illustrated in Fig. 3(a). Therefore, when the grasping sections 3a and 3b are in the closed state, the pair of movement transmission sections 5a and 5b is in contact with each other, and the first link 9 and the second link 10 are arranged to be tilted at a predetermined angle from a direction perpendicular to the longitudinal direction of the movement transmission sections 5a and 5b, and the first link 9 and the second link 10 are formed not to protrude in the width direction of the movement transmission sections 5a and 5b. Further, as illustrated in Fig. 3(b), when the grasping sections 3 a and 3b are in the opened state, the first link 9 and the second link 10 are arranged in a direction approximately perpendicular to the movement transmission sections 5a and 5b, and the first link 9 and the second link 10 are formed not to protrude in the width direction of the movement transmission sections 5a and 5b, and the movement transmission sections 5a and 5b are formed to be slightly separated from each other.

The grasping sections 3a and 3b grasp the medium such as an artificial blood vessel arranged in a living body. A described above, the grasping sections 3a and 3b are opened and closed by being transmitted the opening and closing movement of the handle sections 4a and 4b through the movement transmission sections 5a and 5b. In other words, rotation movement of the one handle section 4b is transmitted as rotation movement of the other grasping section 3 a by the double lever mechanism, so that the grasping sections 3a and 3b are opened and closed.

When the pair of grasping sections 3a and 3b are in the closed state, that is, when the opposing surfaces 8a and 8b in the grasping sections 3a and 3b are in contact with each other, tips of the grasping sections 3a and 3b have a round shape, and the grasping sections 3a and 3b are formed to minimize damage of a biological tissue when the intracorporeal introduction instrument 1 is introduced into a body and the tunnel or the pathway is formed. The shape of the pair of grasping sections 3a and 3b when the grasping sections 3a and 3b are in the closed state is not especially limited as long as the grasping sections 3a and 3b are less likely to damage the biological tissue with the shape when the intracorporeal introduction instrument 1 is introduced into the body. The shape of the pair of grasping sections 3a and 3b when the grasping sections 3a and 3b are in the closed state may be formed to gradually become narrower from the pair of movement transmission sections 5a and 5b toward the tips of the pair of grasping sections 3a and 3b, or may be formed to have an unchanged thickness up to a predetermined position from the pair of movement transmission sections 5a and 5b toward the tips of the pair of grasping sections 3a and 3b, or to become narrow toward the tips after gradually becoming thicker. An external surface of the pair of grasping sections 3a and 3b when the grasping sections 3a and 3b become narrower or thicker toward the tips may be any of a tapered shape, an outwardly bent shape, or an inwardly bent shape. To be specific, examples of the external shape include a truncated shape such as a truncated cone shape or a truncated pyramid shape, having a base end surface of the pair of grasping sections 3a and 3b, that is, a surface perpendicular to the longitudinal direction, as a base, a shape obtained by dividing a spheroid, a globe, or the like into half, a shape that is a combination of the aforementioned shapes, and a duckbill shape that looks like a bill of duck. In any shape, it is favorable that the external surface has no acute angles, and is rounded.

The sizes of the grasping sections 3a and 3b are appropriately set according to a portion used of the intracorporeal introduction instrument 1 or the like, and the thickness may be the same as that of the movement transmission sections 5a and 5b, or may have a thicker portion than the movement transmission sections 5a and 5b. The sizes of the opposing surfaces 8a and 8b in the grasping sections 3a and 3b may just be able to grasp the medium such as an artificial blood vessel. As illustrated in Figs. 2(a) and 2(b), a length L2 of the intracorporeal introduction instrument 1 in the longitudinal direction is favorably 10 to 20 mm, for example, 15 mm. A length D2 of two directions at base end portions of the grasping sections 3a and 3b in the width direction perpendicular to the longitudinal direction is favorably 7 to 13 mm, for example, 10 mm. Further, a total thickness D3 of the pair of grasping sections 3a and 3b at a position of 20% of the length L2 from the tips of the grasping sections 3a and 3b to the base end side, when the pair of grasping sections 3a and 3b are in the closed state, is favorably 4 to 8 mm, for example, 6 mm. A length D4 in a direction perpendicular to the total thickness D3 at the position is favorably 5 to 11 mm, for example, 8 mm.

The opposing surfaces 8a and 8b may be a plane, or unevenness may be formed, which enables the opposing surfaces 8a and 8b to reliably grasp the medium with adequate friction force without damaging the medium when grasping the medium such as an artificial blood vessel.

Next, a method of forming a tunnel in a lower limb, using the intracorporeal introduction instrument 1, and arranging an artificial blood vessel in the tunnel will be described as an example of a method of using the intracorporeal introduction instrument 1 of the present embodiment.

First, portions corresponding to an inlet and an outlet for introducing the intracorporeal introduction instrument 1 are formed in the lower limb. An operator inserts fingers into the ring sections 6a and 6b, holds the intracorporeal introduction instrument 1, introduces the grasping sections 3a and 3b through the inlet in the closed state of the grasping sections 3a and 3b, and inserts the grasping sections 3a and 3b into the outlet, to create the tunnel or the pathway for arranging the artificial blood vessel in a subcutaneous of the lower limb.

Next, the operator applies force in a direction into which the pair of handle sections 4a and 4b are separated, in a state where the intracorporeal introduction instrument 1 is inserted into the lower limb, rotates the one handle section 4b around the first pin 11 in the direction into which the one handle section 4b is separated from the other handle section 4a, and keeps the grasping sections 3a and 3b in the opened state through the double lever mechanism formed by the movement transmission sections 5a and 5b.

As illustrated in Figs. 3(a) and 3(b), in the double lever mechanism, the first link 9 arranged in a tilted manner is rotated around the first pin 11 to get close to the direction perpendicular to the longitudinal direction of the movement transmission sections 5a and 5b, with the rotation of the one handle section 4b. Further, the other movement transmission section 5a connected with the first link 9 through the second pin 12 swings, with the rotation of the first link 9. Further, the second link 10 is rotated around the fourth pin 14 to get close to the direction perpendicular to the longitudinal direction of the movement transmission sections 5a and 5b, with the swing of the other movement transmission section 5a. As a result, the other grasping section 3a integrally connected with the second link 10 is rotated around the fourth pin 14 in the direction being away from the one grasping section 3b. When the grasping sections 3a and 3b are kept in the opened state, the pair of movement transmission sections 5a and 5b are slightly separated from each other. However, the distance therebetween is about 0.1 to several mm, and the external form thereof is almost unchanged.

Next, the operator arranges the end portion of the artificial blood vessel between the pair of grasping sections 3a and 3b, and applies force in a direction into which the pair of handle sections 4a and 4b is adjacent to each other, so that the grasping sections 3a and 3b are closed through the double lever mechanism formed by the movement transmission sections 5a and 5b, and the artificial blood vessel is grasped with the grasping sections 3a and 3b with adequate pressure.

Next, the operator pulls back the intracorporeal introduction instrument 1 in a state where the artificial blood vessel is grasped with the grasping sections 3a and 3b, and inserts the artificial blood vessel into the formed tunnel or pathway. When the artificial blood vessel reaches the inlet formed in the body surface, the operator operates the handle sections 4a and 4b to open the grasping sections 3a and 3b, and releases the artificial blood vessel. In this way, the artificial blood vessel is arranged in the formed tunnel or pathway. Following that, the artificial blood vessel and the biological blood vessel are sutured, and the inlet and the outlet formed in the body surface are sutured, by a commonly-performed method.

In these series of operations, operations such as removing the cap provided on the tip, and fixing the artificial blood vessel with a string are not necessary like the conventional so-called tunneler, after the intracorporeal introduction instrument 1 is introduced into the living body and the tunnel is formed. In these operations, the artificial blood vessel can be inserted into the body by simply grasping and pulling back the artificial blood vessel with the grasping sections. Therefore, the surgical operation can be simplified and the surgical time can be shortened, whereby the burden on the patient and the medical professional can be reduced.

Further, the conventional so-called tunneler is formed of a plurality of parts including a main body, a cap, a string, and the like, and each of the parts is attachably/detachably formed. Therefore, management of the parts is complicated. However, the intracorporeal introduction instrument 1 does not require attachment/detachment of a plurality of parts at the time of use, and the structure is simple. Therefore, washing and sterilization are easy, and the instrument can be easily managed. Therefore, according to the present invention, management of medical instruments can be rationalized, and total cost can be decreased, accordingly.

Next, a second embodiment of an intracorporeal introduction instrument of the present invention will be described.
Fig. 4(a) is a top-view schematic explanatory diagram of an intracorporeal introduction instrument, illustrating an embodiment of an intracorporeal introduction instrument according to the present invention, and Fig. 4(b) is a side-view schematic explanatory diagram of an intracorporeal introduction instrument, illustrating an embodiment of an intracorporeal introduction instrument according to the present invention.

An intracorporeal introduction instrument 20 of an embodiment illustrated in Figs. 4(a) and 4(b) is similar to the intracorporeal introduction instrument 1 illustrated in Figs. 1(a) and 1(b), except that an opening and closing direction of handle sections 41a and 41b, and an opening and closing direction of grasping sections 31a and 31b are perpendicular to each other.

In the intracorporeal introduction instrument 20 of the present embodiment, the opening and closing direction of the handle sections 41a and 41b, and the opening and closing direction of the grasping sections 31a and 31b are perpendicular to each other. As illustrated in Figs. 4(a) and 4(b), when the handle sections 41a and 41b perform opening and closing movement along the paper surface, that is, when the handle section 41b is rotated around a first pin 111 along the paper surface, the grasping sections 31a and 31b are opened and closed in a direction perpendicular to the paper surface. That is, the grasping section 31a rotates along the surface perpendicular to the paper surface centering around the fourth pin 141.

The thickness of movement transmission sections 51a and 51b of the intracorporeal introduction instrument 20 of the present embodiment is changed in the middle thereof. When the intracorporeal introduction instrument 20 is viewed from a top view, as illustrated in Fig. 4(a), the movement transmission sections 51a and 51b at the handle sections 41 a and 41b side have a width on the same level with arm sections 71a and 71b, and the width becomes gradually larger toward the grasping sections 31a and 31b, and becomes a width on the same level with the grasping sections 31a and 31b at the grasping sections 31a and 31b side. When the intracorporeal introduction instrument 20 is viewed from a side view, as illustrated in Fig. 4(b), the movement transmission sections 51a and 51b at the handle sections 41a and 41b side have a height on the same level with the arm sections 71a and 71b, and the height becomes half in the middle thereof, and becomes a height on the same level with the grasping section at the grasping sections 31a and 31b side. Further, as illustrated in Fig. 4(a), at the handle sections 41a and 41b side, the movement transmission section 51a and the movement transmission section 51b are arranged in parallel along the paper surface. Meanwhile, at the grasping sections 31a and 31b side, the movement transmission section 51a and the movement transmission section 51b are arranged to overlap each other in the direction perpendicular to the paper surface.

In the intracorporeal introduction instrument 20 of the present embodiment, when the handle section 41b is rotated in a direction being away from the handle section 41a, the movement transmission section 51a is moved to the handle section 41a side with respect to the movement transmission section 51b, and the movement transmission section 51a is moved in a direction being slightly away from the movement transmission section 51b That is, when the handle sections 41a and 41b are caused to be in an opened state in such a manner that an angle between the handle section 41a and the handle section 41b becomes large, the movement transmission section 51a and the movement transmission section 51b are slightly separated at the grasping sections 31a and 31b side, and the movement transmission section 51a is moved to the handle section 41a side, so that the grasping section 31a is rotated around a fourth pin 141, and the grasping sections 31a and 31b become in the opened state.

When the intracorporeal introduction instrument 20 is formed such that the opening and closing direction of the handle sections 41a and 41b and the opening and closing direction of the grasping sections 31a and 31b are perpendicular to each other, the intracorporeal introduction instrument 20 can be inserted into an inlet formed in a body surface, in a posture where the handle sections 41a and 41b can be opened and closed along a body surface. Therefore, the intracorporeal introduction instrument 20 can be introduced to thread the vicinity of the subcutaneous, to form the tunnel or the pathway. When the handle sections 41a and 41b are operated and caused to be in the opened state, after the grasping sections 31a and 31b are led through the outlet formed in the body surface, the grasping section 3a is rotated in a direction rising from the body surface, and the grasping sections 3a and 3b are caused to be in the opened state. The grasping sections 3a and 3b grasp a medium such as an artificial blood vessel to be inserted into the tunnel or the pathway, and the intracorporeal introduction instrument 20 is pulled back, so that the medium such as an artificial blood vessel can be arranged in the formed tunnel or pathway.

Next, a third embodiment of an intracorporeal introduction instrument of the present embodiment will be described.
Fig. 5(a) is a top-view schematic explanatory diagram of an intracorporeal introduction instrument, illustrating another embodiment of an intracorporeal introduction instrument according to the present invention, and Fig. 5(b) is a side-view schematic explanatory diagram of an intracorporeal introduction instrument, illustrating an embodiment of an intracorporeal introduction instrument according to the present invention.

An intracorporeal introduction instrument 30 of an embodiment illustrated in Figs. 5(a) and 5(b) is similar to the intracorporeal introduction instrument 1 illustrated in Figs. 1(a) and 1(b), except that movement transmission sections 52a and 52b have a gently bent curved section 15. In Figs. 5(a) and 5(b), a member having a similar shape to the member illustrated in Figs. 1(a) and 1(b) is illustrated using the same reference sign as that illustrated in Figs. 1(a) and 1(b).

As illustrated in Fig. 5(a), the movement transmission sections 52a and 52b in the intracorporeal introduction instrument 30 of the present embodiment have the curved section 15 gently bent in a direction perpendicular to an opening and closing direction of handle sections 4a and 4b. If the movement transmission sections 52a and 52b have such a curved section 15, grasping sections 32a and 32b can be easily inserted into the vicinity of a subcutaneous and exposed through an outlet formed in a body surface, after the intracorporeal introduction instrument 30 is introduced through an inlet formed in the body surface. Therefore, the intracorporeal introduction instrument 30 can be introduced in the vicinity of the subcutaneous without applying excessive force. Therefore, damage to a biological tissue can be minimized.

A bending angle of the curved section 15 is appropriately set according to the depth and the length of a tunnel formed in a living body, and is appropriately set within a range of 100 to 175°, for example. Further, it is favorable that a curvature radius is appropriately set within a range of 500 to 1500 mm.

Note that the intracorporeal introduction instrument of the present invention is not limited to the above-described embodiments, and various changes can be made as long as the object of the present invention can be achieved.

For example, the dimensions of the respective sections of the intracorporeal introduction instrument 1 have been exemplarily described in the above embodiments. However, the dimensions of the intracorporeal introduction instrument of the present invention can be appropriately set according to the portion used the like. For example, when the intracorporeal introduction instrument is introduced into an arm and is used to form a tunnel and enable a medium such as the artificial blood vessel to be inserted into, dimensions of an intracorporeal introduction instrument can be about half of the dimensions described in the intracorporeal introduction instrument 1.

The intracorporeal introduction instrument of the present invention is used to arrange a medium such as an artificial blood vessel in a formed tunnel or pathway, by being introduced into a living body, forming the tunnel or the pathway, and grasping and pulling back the medium such as an artificial blood vessel, and is especially favorably used in treatment of a varix caused in a lower limb.

### Reference Signs List

1, 20, and 30 Intracorporeal introduction instrument
2 Rod-like member
3a, 3b, 31a, 31b, 32a, and 32b Grasping section
4a, 4b, 41a, and 41b Manipulation section
5a, 5b, 52a, and 52b Movement transmission section
6a and 6b Ring section
7a, 7b, 71a, and 71b Arm section
8a and 8b Opposing surface
9 First link
10 Second link
11 and 111 First pin
12 and 121 Second pin
13 and 131 Third pin
14 and 141 Fourth pin
15 Curved section

## Claims

1. An intracorporeal introduction instrument comprising:
grasping sections at one end of a pair of rod-like members;
handle sections at the other end of the pair of rod-like members; and
movement transmission sections between the grasping sections and the handle sections,
the grasping sections configured to be opened and closed through the movement transmission sections according to opening and closing of the handle sections, wherein
the movement transmission sections form a double lever mechanism that transmits opening and closing movement of the handle sections, as opening and closing movement of the grasping sections.

2. The intracorporeal introduction instrument according to claim 1, wherein
an opening and closing direction of the handle sections and an opening and closing direction of the grasping sections are perpendicular to each other.

3. The intracorporeal introduction instrument according to claim 1 or 2, wherein
the movement transmission sections include a gently bent curved section.

4. The intracorporeal introduction instrument according to any one of claims 1 to 3, wherein
an external form of the grasping sections is an approximately truncated pyramid shape when the pair of grasping sections is in a closed state.

5. The intracorporeal introduction instrument according to any one of claims 1 to 4, wherein
a length L1 of the movement transmission sections in a longitudinal direction is 170 to 350 mm, a length L2 of the grasping sections in the longitudinal direction is 10 to 20 mm, a length D1 of the movement transmission sections in a width direction perpendicular to the longitudinal direction is 7 to 13 mm, a length D2 at base end portions of the grasping sections in the width direction is 7 to 13 mm, a total thickness D3 at a position of 20% of the length L2 from a tip of the grasping sections of when the pair of grasping sections is in the closed state is 4 to 8 mm, and a length D4 in a direction perpendicular to the total thickness D3 at the position is 5 to 11 mm.
